(19) Europäisches Patentamt
European Patent Office
Office européen des brevets

(11) EP 4 212 182 A2

(12) EUROPEAN PATENT APPLICATION

(43) Date of publication:
19.07.2023 Bulletin 2023/29

(21) Application number: 22210789.8

(22) Date of filing: 01.12.2022

(51) International Patent Classification (IPC):
*A61L 2/08* (2006.01)

(52) Cooperative Patent Classification (CPC):
A61L 2/081

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 22.12.2021 JP 2021208609

(71) Applicant: Sumitomo Rubber Industries, Ltd.
Kobe-shi, Hyogo 651-0072 (JP)

(72) Inventors:
- KIDA, Yogun
Kobe-shi, Hyogo, 651-0072 (JP)
- TAJIMA, Kei
Kobe-shi, Hyogo, 651-0072 (JP)
- NOJIRI, Kazuki
Kobe-shi, Hyogo, 651-0072 (JP)
- MATSUTANI, Yuichiro
Kobe-shi, Hyogo, 651-0072 (JP)
- ONISHI, Toshiki
Kobe-shi, Hyogo, 651-0072 (JP)
- KONDO, Toshikazu
Kobe-shi, Hyogo, 651-0072 (JP)

(74) Representative: Manitz Finsterwald
Patent- und Rechtsanwaltspartnerschaft mbB
Martin-Greif-Strasse 1
80336 München (DE)

# (54) STERILIZATION METHOD FOR MEDICAL RUBBER PART

(57) An object of the present disclosure is to provide a sterilization method for a medical rubber part in which non-elution characteristics are maintained even after sterilization with gamma ray and which experiences less troubles in a manufacturing process for the medical product. The sterilization method for a medical rubber part includes irradiating, with gamma ray, a packaging article for a medical rubber part made from an elastomer that contains a polyethylene, the packaging article accommodating a plurality of the medical rubber parts.

EP 4 212 182 A2

## Description

BACKGROUND OF THE INVENTION

Field of the Invention

The present disclosure relates to a sterilization method for a medical rubber part.

Background Art

Medical rubber plugs for sealing an opening of a syringe, a vial, or the like are required to have many characteristics such as non-elution characteristics, high cleanability, chemical resistance, resistance to needle piercing, self-sealability, and high slidability. Quality characteristics that are required of the medical rubber plugs should, in terms of use of the medical rubber plugs, comply with the regulations stipulated in "Test for Rubber Closure for Aqueous Infusions" of the 17th edition of the Japanese Pharmacopoeia.

For example, Japanese Laid-Open Patent Publication No. H10-179690 discloses a rubber plug for a pharmaceutical agent container, the rubber plug being obtained by blending 5 to 25 parts by weight of fine powder of ultrahigh-molecular-weight polyethylene per 100 parts by weight of halogenated isobutylene-isoprene rubber, and vulcanizing the resultant halogenated isobutylene-isoprene rubber by using at least one of 2-substituted-4,6-dithiol-s-triazine derivatives or by using an organic peroxide, in the absence of a zinc compound.

There is an increasing demand for medical rubber products (syringe gaskets, vial plugs, and the like) to be delivered in a state of guaranteeing sterilization thereof, i.e., to be ready-to-use (RTU). Examples of a method for guaranteeing sterilization include methods involving sterilization with high-pressure steam, sterilization with ethylene oxide gas (EOG), and sterilization with gamma ray. The method involving sterilization with gamma ray has an advantage that a medical rubber product can be sterilized while being packaged and thus can be delivered without opening the package. Meanwhile, the method involving sterilization with EOG has environment-related issues. Considering this, the method for guaranteeing sterilization tends to be switched to the method involving sterilization with gamma ray.

The method involving sterilization with gamma ray guarantees sterilization by means of absorbed dose setting and actually measured values. If a plurality of medical rubber parts are packed into a packaging bag and sterilization with gamma ray is performed, unevenness among the medical rubber parts might occur in the packaging bag. Thus, even when the packaging bag is irradiated with a predetermined radiation dose of gamma ray, variation in the absorbed dose of gamma ray occurs in the packaging bag. This gives rise to: medical rubber parts having low absorbed doses of gamma ray; and medical rubber parts having high absorbed doses of gamma ray. However, it is necessary to ensure, for each medical rubber part, a minimum absorbed dose with which the medical rubber part can be sterilized. Thus, it is necessary to irradiate the packaging bag with at least the minimum absorbed dose of gamma ray. This gives rise to medical rubber parts that absorb an excessive dose of gamma ray at the time of sterilization with gamma ray, in the packaging bag.

Japanese Laid-Open Patent Publication No. 2002-301133 discloses: a rubber composition containing an isobutylene copolymer as a main component and having a density not higher than 0.95, the rubber composition being used for a medical rubber plug or a medical rubber product on which radiation treatment is easily performed; and a crosslinked product of the rubber composition.

Japanese Laid-Open Patent Publication (Translation of PCT Application) No. 2017-531604 discloses a method for packaging a part (1), made from an elastomer, such as a plug for a pharmaceutical agent container. The method includes: a step of packing the part (1) into a primary bag (10) made from a material substantially impermeable with air; and a step of applying an atmosphere with at least 80% of nitrogen to the inside of the primary bag (10). In the method, the primary bag (10) is put in a secondary bag (20), and the interval between the primary bag (10) and the secondary bag (20) is set to be in a vacuum state.

When a medical rubber part is sterilized by being irradiated with gamma ray, cleavage and crosslinking simultaneously occurs in a polymer forming the medical rubber part. If an excessive dose of gamma ray is absorbed, cleavage of the main chain of the polymer forming the medical rubber part is promoted, whereby low-molecular-weight components are generated. Consequently, the non-elution characteristics of the medical rubber part having been subjected to sterilization with gamma ray deteriorates. In addition, bleed-out, onto a surface of the rubber part, of the low-molecular-weight components resulting from the cleavage occurs, and medical rubber parts come into close contact with each other. Consequently, a trouble of clogging in a parts feeder used in a manufacturing process for medical products occurs.

It is also conceivable to blend an antioxidant to the medical rubber part in preparation for absorption of an excessive dose of gamma ray. However, addition of an antioxidant leads to concerns that: non-elution characteristics decrease; and a medical preparation is adversely influenced.

## SUMMARY OF THE INVENTION

The present disclosure has been made in view of the above circumstances, and an object of the present disclosure is to provide a sterilization method for a medical rubber part in which non-elution characteristics are maintained even after sterilization with gamma ray and which experiences less troubles in a manufacturing process for the medical product.

A sterilization method for a medical rubber part of the present disclosure includes irradiating, with gamma ray, a packaging article for a medical rubber part made from an elastomer that contains a polyethylene, the packaging article accommodating a plurality of the medical rubber parts.

The present disclosure makes it possible to provide a sterilization method for a medical rubber part in which non-elution characteristics are maintained even after sterilization with gamma ray and which experiences less troubles in a manufacturing process for the medical product.

## BRIEF DESCRIPTION OF THE DRAWINGS

FIG. 1 is a diagram for schematically explaining an exemplary packaging mode for a medical rubber part of the present disclosure;
FIG. 2 is a diagram for schematically explaining another exemplary packaging mode for the medical rubber part of the present disclosure; and
FIG. 3 is a diagram for schematically explaining a tack test method for the medical rubber part of the present disclosure.

## DETAILED DESCRIPTION

A sterilization method for a medical rubber part of the present disclosure includes irradiating, with gamma ray, a packaging article for a medical rubber part made from an elastomer that contains a polyethylene, the packaging article accommodating a plurality of the medical rubber parts.

Examples of the gamma ray can include gamma rays emitted from cobalt-60, cesium-137, and the like. Gamma ray emitted from cobalt-60 is preferable.

Regarding irradiation with the gamma ray, an absorbed dose of the gamma ray for the medical rubber part is set through an actual sterilization validation procedure. For ordinary medical devices, operation is performed with a minimum absorbed dose being set to 15 kGy in many cases. A radiation dose, of the gamma ray, at which the absorbed doses of the gamma ray in all of the medical rubber parts in the packaging article take values not lower than 15 kGy, varies depending on the number of the medical rubber parts in the packaging article, the manner in which the medical rubber parts are put into the packaging article, and the like. In general, irradiation is performed in a dose that falls within a range of not lower than 1.4 times 15 kGy and not higher than 2.0 times 15 kGy. Likewise, if the minimum absorbed dose is set to 20 kGy, irradiation is performed in a dose that falls within a range of not lower than 1.4 times 20 kGy and not higher than 2.0 times 20 kGy, and, if the minimum absorbed dose is set to 25 kGy, irradiation is performed in a dose that falls within a range of not lower than 1.4 times 25 kGy and not higher than 2.0 times 25 kGy. The absorbed dose of the gamma ray can be ascertained by attaching a dosemeter to an object that is to be irradiated.

The packaging article for accommodating the medical rubber parts not having yet been irradiated with the gamma ray has an oxygen concentration that is preferably not higher than 5%, more preferably not higher than 3%, and further preferably not higher than 1%. The reason for this is because, if the oxygen concentration in the packaging article is set to be not higher than 5%, degradation of each medical rubber part due to irradiation with the gamma ray can be suppressed.

Examples of a method for setting the oxygen concentration in the packaging article to be not higher than 5% can include: a method in which air in the packaging article is substituted with an inert gas; and a method in which an oxygen adsorber is accommodated in the packaging article.

Examples of the inert gas can include: rare gases such as helium gas, neon gas, and argon gas; nitrogen gas; and the like.

Examples of the oxygen adsorber can include AGELESS (commercially available product) which is an iron-based oxygen adsorber, and the like.

The packaging article for accommodating the medical rubber part is not particularly limited as long as the packaging article allows irradiation with the gamma ray. Examples of the form of the packaging article can include the forms of a bag, a box, and the like. Examples of the packaging bag can include packaging bags formed of aluminum or a thermoplastic resin film made from polyethylene, polyamide, or polyester. The packaging bag is preferably one that can be sealed. The packaging box is not particularly limited, and examples of the packaging box can include a box made

from paper, a box made from cardboard, and the like.

Examples of the packaging article can include: a packaging article having gas permeability; and a packaging article having non-gas permeability (gas sealability). It is also preferable to use these packaging articles in combination.

Irradiation of the medical rubber part with the gamma ray may be performed on, for example, a packaging article (for example, a cardboard box) further accommodating a plurality of primary packaging articles (for example, packaging bags) accommodating a plurality of the medical rubber parts.

FIG. 1 is a diagram for schematically explaining an exemplary packaging mode for irradiation with the gamma ray. In the mode shown in FIG. 1, a primary packaging article 3 accommodating a plurality of medical rubber parts 1 is further accommodated in a secondary static charge prevention packaging article 5 and a tertiary static charge prevention packaging article 7. As the primary packaging article 3, a packaging article having gas permeability is preferable. As the secondary static charge prevention packaging article 5 and the tertiary static charge prevention packaging article 7, packaging articles capable of sealing gas are preferable. Each of the secondary static charge prevention packaging article 5 and the tertiary static charge prevention packaging article 7 is preferably sealed with a heat seal 9. In the case of using an oxygen adsorber 11, the oxygen adsorber 11 is preferably disposed between the primary packaging article 3 and the secondary packaging article 5 such that the oxygen adsorber 11 does not come into direct contact with the medical rubber parts 1. By disposing the oxygen adsorber 11 in the secondary packaging article 5, the oxygen concentration in each of the secondary packaging article 5 and the primary packaging article 3 can be set to be not higher than 5%. Irradiation with the gamma ray can be performed with a plurality of the tertiary static charge prevention packaging articles 7 being accommodated in a quaternary packaging article (for example, a cardboard box).

FIG. 2 is a diagram for schematically explaining another exemplary packaging mode for irradiation with the gamma ray. In the mode shown in FIG. 2, the primary packaging article 3 accommodating the plurality of medical rubber parts 1 are further accommodated in the secondary static charge prevention packaging article 5 and the tertiary static charge prevention packaging article 7. Each of the secondary static charge prevention packaging article 5 and the tertiary static charge prevention packaging article 7 is preferably sealed with the heat seal 9. As the primary packaging article 3, a packaging article having gas permeability is preferable. As the secondary static charge prevention packaging article 5 and the tertiary static charge prevention packaging article 7, packaging articles capable of sealing gas are preferable. The secondary packaging article 5 accommodating the primary packaging article 3 is filled with an inert gas. The filling with the inert gas makes it possible to set the oxygen concentration in each of the secondary packaging article 5 and the primary packaging article 3 to be not higher than 5%. Irradiation with the gamma ray can be performed with a plurality of the tertiary static charge prevention packaging articles 7 being accommodated in a quaternary packaging article (for example, a cardboard box).

At the time of irradiation with the gamma ray, irradiation with the gamma ray is preferably performed in a state where the packaging article accommodating the plurality of medical rubber parts is accommodated in, for example, an accommodation container made from an aluminum alloy.

Examples of the medical rubber part of the present disclosure include: rubber plugs and sealing members of containers (for example, vials) for various medical preparations such as a liquid preparation, a powder preparation, and a freeze-dried preparation; rubber plugs for vacuum blood collection tubes; slidable parts and sealing parts such as plunger stoppers and nozzle caps for pre-filled syringes; and the like.

The medical rubber part of the present disclosure to be subjected to sterilization treatment is made from an elastomer that contains a polyethylene. The elastomer is preferably a cured product of a medical rubber composition that contains: a (a) base polymer containing a halogenated isobutylene-isoprene rubber; a (b) polyethylene; and a (c) triazine derivative as a crosslinking agent. Hereinafter, raw materials contained in the medical rubber composition of the present disclosure will be described.

Firstly, the (a) base polymer containing a halogenated isobutylene-isoprene rubber will be described. Examples of the halogenated isobutylene-isoprene rubber include: chlorinated isobutylene-isoprene rubber; brominated isobutylene-isoprene rubber; a bromide of a copolymer rubber of isobutylene and p-methylstyrene (brominated isobutylene-para-methylstyrene copolymer rubber); and the like.

As the halogenated isobutylene-isoprene rubber, a chlorinated isobutylene-isoprene rubber or a brominated isobutylene-isoprene rubber is preferable. The chlorinated isobutylene-isoprene rubber or the brominated isobutylene-isoprene rubber is obtained by, for example, causing a reaction in which: chlorine or bromine is added to an isoprene structural moiety (specifically, a double bond and/or a carbon atom adjacent to the double bond) in an isobutylene-isoprene rubber; or the isoprene structural moiety is substituted with chlorine or bromine. The isobutylene-isoprene rubber is a copolymer obtained by polymerizing isobutylene and a small amount of isoprene.

The halogen content of the halogenated isobutylene-isoprene rubber is preferably not lower than 0.5% by mass, more preferably not lower than 1% by mass, and further preferably not lower than 1.5% by mass. Meanwhile, the halogen content is preferably not higher than 5% by mass, more preferably not higher than 4% by mass, and further preferably not higher than 3% by mass.

Specific examples of the chlorinated isobutylene-isoprene rubber include at least one of: CHLOROBUTYL 1066

[stabilizer: NS, halogen content: 1.26%, Mooney viscosity: 38 $ML_{1+8}$ (125°C), specific gravity: 0.92] manufactured by JAPAN BUTYL Co., Ltd.; LANXESS X_BUTYL CB1240 manufactured by LANXESS; and the like.

Specific examples of the brominated isobutylene-isoprene rubber include at least one of: BROMOBUTYL 2255 [stabilizer: NS, halogen content: 2.0%, Mooney viscosity: 46 $ML_{1+8}$ (125°C), specific gravity: 0.93] manufactured by JAPAN BUTYL Co., Ltd.; LANXESS X_BUTYL BBX2 manufactured by LANXESS; and the like.

The (a) base polymer may contain a rubber component other than halogenated isobutylene-isoprene rubber. Examples of the other rubber component include butyl-based rubbers, isoprene rubber, butadiene rubber, styrene-butadiene rubber, natural rubber, chloroprene rubber, nitrile-based rubbers such as acrylonitrile-butadiene rubber, hydrogenated nitrile-based rubbers, norbornene rubber, ethylene-propylene rubber, ethylene-propylene-diene rubber, acrylic rubber, ethylene-acrylate rubber, fluororubber, chlorosulfonated polyethylene rubber, epichlorohydrin rubber, silicone rubber, urethane rubber, polysulfide rubber, phosphazene rubber, 1,2-polybutadiene, and the like. These rubber components may be used singly, or two or more of these rubber components may be used in combination.

In the case of using the other rubber component, the proportion of the halogenated isobutylene-isoprene rubber contained in the (a) base polymer is preferably not lower than 90% by mass, more preferably not lower than 95% by mass, and further preferably not lower than 98% by mass. A mode in which the (a) base polymer contains only the halogenated isobutylene-isoprene rubber is also preferable.

The medical rubber composition of the present disclosure contains the (b) polyethylene. The polyethylene is more likely to absorb gamma ray than the (a) base polymer is. Thus, the polyethylene has an effect of preventing cleavage of chains of the (a) base polymer due to irradiation with the gamma ray. In addition, a polyethylene having a low degree of crystallinity has a branched chain, and it is considered that crosslinking progresses without cleavage of the main chain even upon irradiation with the gamma ray. As a result, the non-elution characteristic of the medical rubber composition is considered to be improved.

From this viewpoint, examples of the (b) polyethylene to be used in the present disclosure can include high-density polyethylene (HDPE) and low-density polyethylene (LDPE). The high-density polyethylene (HDPE) and the low-density polyethylene (LDPE) may be used singly, or the high-density polyethylene (HDPE) and the low-density polyethylene (LDPE) may be used in combination.

In the case of using the high-density polyethylene (HDPE) and the low-density polyethylene (LDPE) in combination, the mass ratio (HDPE/LDPE) of the high-density polyethylene (HDPE) to the low-density polyethylene (LDPE) is preferably not lower than 0.3, more preferably not lower than 0.5, and further preferably not lower than 1.0. Meanwhile, the mass ratio (HDPE/LDPE) is preferably not higher than 5.0, more preferably not higher than 4.0, and further preferably not higher than 3.0. The reason for this is because, if the mass ratio (HDPE/LDPE) of the high-density polyethylene (HDPE) to the low-density polyethylene (LDPE) falls within the aforementioned range, a radical absorption effect at the time of irradiation with the gamma ray and an appropriate hardness of the rubber can be ensured.

The (b) polyethylene preferably contains a polyethylene having a degree of crystallinity not higher than 70%.

The degree of crystallinity of the high-density polyethylene (HDPE) is preferably 60% to 80%, more preferably 60% to 75%, and further preferably 60% to 70%. The degree of crystallinity of the low-density polyethylene (LDPE) is preferably 30% to 50%, more preferably 30% to 45%, and further preferably 30% to 40%. The reason for this is because, if the degree of crystallinity of the polyethylene falls within the aforementioned range, radicals generated by irradiation with the gamma ray are effectively absorbed, and cleavage of the main chain of the polymer is prevented.

The degree of crystallinity of the (b) polyethylene is determined according to the following expression.

$$\text{Degree of crystallinity (\%)} = (\text{measured melting heat quantity (J/g)}/\text{perfect crystal melting heat quantity (J/g)}) \times 100$$

The perfect crystal melting heat quantity (J/g) is 293 J/g (a value in a literature) and is a melting heat quantity of the polyethylene at 100%-crystallinity. A measurement method for the melting heat quantity of the polyethylene will be described later.

As the (b) polyethylene, the low-density polyethylene is preferable. The density (g/cm$^3$) of the high-density polyethylene is preferably 0.930 to 0.960 and more preferably 0.930 to 0.950. The density (g/cm$^3$) of the low-density polyethylene is not particularly limited, but is preferably 0.910 to 0.925 and more preferably 0.910 to 0.920.

As the (b) polyethylene, a polyethylene in the form of fine powder is preferably used. The volume-average particle diameter of the polyethylene in the form of fine powder is preferably not smaller than 10 μm, more preferably not smaller than 15 μm, and further preferably not smaller than 20 μm. Meanwhile, the volume-average particle diameter is preferably not larger than 200 μm, more preferably not larger than 160 μm, and further preferably not larger than 120 μm. The reason for this is because, if the particle diameter of the polyethylene in the form of fine powder falls within the aforementioned range, it becomes easy for the polyethylene to be evenly mixed or dispersed in the polymer.

The blending amount of the (b) polyethylene per 100 parts by mass of the (a) base polymer is preferably not smaller than 3 parts by mass, more preferably not smaller than 5 parts by mass, and further preferably not smaller than 10 parts by mass. Meanwhile, the blending amount is preferably not larger than 30 parts by mass, more preferably not larger than 25 parts by mass, and further preferably not larger than 20 parts by mass. The reason for this is because, if the blending amount of the (b) polyethylene falls within the aforementioned range, radicals generated at the time of irradiation with the gamma ray can be effectively absorbed, and cleavage of the main chain of the polymer can be prevented.

The medical rubber composition of the present disclosure preferably contains a triazine derivative as the (c) crosslinking agent.

The triazine derivative acts as a crosslinking agent on the halogenated isobutylene-isoprene rubber. Examples of the triazine derivative include a compound represented by a general formula (1).

[Chem. 1]

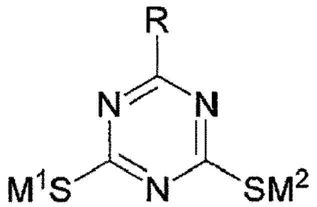

(1)

[In the formula, R represents -SH, $-OR^1$, $-SR^2$, $-NHR^3$, or $-NR^4R^5$ ($R^1$, $R^2$, $R^3$, $R^4$, and $R^5$ each represent an alkyl group, an alkenyl group, an aryl group, an aralkyl group, an alkylaryl group, or a cycloalkyl group. $R^4$ and $R^5$ may be identical to each other or different from each other.). $M^1$ and $M^2$ each represent H, Na, Li, K, 1/2Mg, 1/2Ba, 1/2Ca, an aliphatic primary, secondary, or tertiary amine, a quaternary ammonium salt, or a phosphonium salt. $M^1$ and $M^2$ may be identical to each other or different from each other.]

In the general formula (1), examples of the alkyl group include alkyl groups having 1 to 12 carbon atoms, such as a methyl group, an ethyl group, an n-propyl group, an isopropyl group, an n-butyl group, an isobutyl group, a tert-butyl group, an n-pentyl group, an isopentyl group, a tert-pentyl group, an n-hexyl group, a 1,1-dimethylpropyl group, an octyl group, an isooctyl group, a 2-ethylhexyl group, a decyl group, and a dodecyl group. Examples of the alkenyl group include alkenyl groups having 1 to 12 carbon atoms, such as a vinyl group, an allyl group, a 1-propenyl group, an isopropenyl group, a 2-butenyl group, a 1,3-butadienyl group, and a 2-pentenyl group. Examples of the aryl group include monocyclic aromatic hydrocarbon groups and condensed polycyclic aromatic hydrocarbon groups, and examples thereof include: aryl groups having 6 to 14 carbon atoms, such as a phenyl group, a naphthyl group, an anthryl group, a phenanthryl group, and an acenaphthylenyl group; and the like. Examples of the aralkyl group include aralkyl groups having 7 to 19 carbon atoms, such as a benzyl group, a phenethyl group, a diphenylmethyl group, a 1-naphthylmethyl group, a 2-naphthylmethyl group, a 2,2-diphenylethyl group, a 3-phenylpropyl group, a 4-phenylbutyl group, a 5-phenylpentyl group, a 2-biphenylylmethyl group, a 3-biphenylylmethyl group, and a 4-biphenylylmethyl group. Examples of the alkylaryl group include alkylaryl groups having 7 to 19 carbon atoms, such as a tolyl group, a xylyl group, and an octylphenyl group. Examples of the cycloalkyl group include: cycloalkyl groups having 3 to 9 carbon atoms, such as a cyclopropyl group, a cyclobutyl group, a cyclopentyl group, a cyclohexyl group, a cycloheptyl group, a cyclooctyl group, and a cyclononyl group; and the like.

Specific examples of the triazine derivative represented by the general formula (1) include 2,4,6-trimercapto-s-triazine, 2-methylamino-4,6-dimercapto-s-triazine, 2-(n-butylamino)-4,6-dimercapto-s-triazine, 2-octylamino-4,6-dimercapto-s-triazine, 2-propylamino-4,6-dimercapto-s-triazine, 2-diallylamino-4,6-dimercapto-s-triazine, 2-dimethylamino-4,6-dimercapto-s-triazine, 2-dibutylamino-4,6-dimercapto-s-triazine, 2-di(iso-butylamino)-4,6-dimercapto-s-triazine, 2-dipropylamino-4,6-dimercapto-s-triazine, 2-di(2-ethylhexyl)amino-4,6-dimercapto-s-triazine, 2-dioleylamino-4,6-dimercapto-s-triazine, 2-laurylamino-4,6-dimercapto-s-triazine, 2-anilino-4,6-dimercapto-s-triazine, and sodium salts and disodium salts thereof.

Among these triazine derivatives, 2,4,6-trimercapto-s-triazine, 2-dialkylamino-4,6-dimercapto-s-triazine, and 2-anilino-4,6-dimercapto-s-triazine are preferable, and 2-dibutylamino-4,6-dimercapto-s-triazine is particularly preferable since 2-dibutylamino-4,6-dimercapto-s-triazine is easy to obtain.

Other examples of the triazine derivative include one or more of 6-[bis(2-ethylhexyl)amino]-1,3,5-triazine-2,4-dithiol, 6-diisobutylamino-1,3,5-triazine-2,4-dithiol, 6-dibutylamino-1,3,5-triazine-2,4-dithiol, 6-dibutylamino-1,3,5-triazine-2,4-dithiol monosodium, 6-anilino-1,3,5-triazine-2,4-dithiol, 1,3,5-triazine-2,4,6-trithiol, and the like.

In the present disclosure, these triazine derivatives may be used singly, or two or more of these triazine derivatives may be used in combination.

In the medical rubber composition of the present disclosure, the amount of the (c) triazine derivative contained per 100 parts by mass of the (a) base polymer component is preferably not smaller than 0.1 parts by mass, more preferably not smaller than 0.3 parts by mass, and further preferably not smaller than 0.5 parts by mass. Meanwhile, the amount is preferably not larger than 2.0 parts by mass, more preferably not larger than 1.4 parts by mass, and further preferably not larger than 1.2 parts by mass. The reason for this is because, if the amount of the (c) triazine derivative falls within the aforementioned range, a rubber having favorable rubber physical properties (hardness, tensile properties, Cset) and good non-elution characteristic and processability (less susceptibility to scorching) can be obtained.

The medical rubber composition of the present disclosure preferably contains no vulcanization accelerator. The reason for this is because a vulcanization accelerator could remain in a rubber product obtained as a final product and could elute into a drug solution inside a syringe or a vial. Examples of the vulcanization accelerator include guanidine-based accelerators (e.g., diphenylguanidine), thiuram-based accelerators (e.g., tetramethylthiuram disulfide and tetramethylthiuram monosulfide), dithiocarbamate-based accelerators (e.g., zinc dimethyldithiocarbamate), thiazole-based accelerators (e.g., 2-mercaptobenzothiazole and dibenzothiazyl disulfide), and sulfenamide-based accelerators (N-cyclohexyl-2-benzothiazole sulfenamide and N-t-butyl-2-benzothiazole sulfenamide).

The medical rubber composition of the present disclosure may contain a hydrotalcite. The hydrotalcite functions as an anti-scorching agent upon crosslinking in the halogenated isobutylene-isoprene rubber and also has a function of preventing increase in permanent strain upon compression in the medical rubber part. Further, the hydrotalcite functions also as an acid acceptor for absorbing chlorine-based gas and bromine-based gas, which have been generated upon crosslinking in the halogenated isobutylene-isoprene rubber, and preventing occurrence of, for example, crosslinking inhibition due to these gases. Magnesium oxide can also function as an acid acceptor.

Examples of the hydrotalcite include one or more of Mg-Al-based hydrotalcites such as $Mg_{4.5}Al_2(OH)_{13}CO_3 \cdot 3.5H_2O$, $Mg_{4.5}Al_2(OH)_{13}CO_3$, $Mg_4Al_2(OH)_{12}CO_3 \cdot 3.5H_2O$, $Mg_6Al_2(OH)_{16}CO_3 \cdot 4H_2O$, $Mg_5Al_2(OH)_{14}CO_3 \cdot 4H_2O$, and $Mg_3Al_2(OH)_{10}CO_3 \cdot 1.7H_2O$, and the like.

Specific examples of the hydrotalcite include DHT-4A (registered trademark)-2 manufactured by Kyowa Chemical Industry Co., Ltd., and the like.

In the case where a hydrotalcite is used as an acid acceptor in the medical rubber composition of the present disclosure, the hydrotalcite is preferably used in combination with MgO. In this case, the blending amount of the hydrotalcite is preferably considered in terms of the total amount of the acid acceptors (hydrotalcite and MgO). The total amount of the acid acceptors (hydrotalcite and MgO) contained per 100 parts by mass of the (a) base polymer component is preferably not smaller than 0.5 parts by mass and more preferably not smaller than 1 part by mass. Meanwhile, the total amount is preferably not larger than 15 parts by mass and more preferably not larger than 10 parts by mass. The reason for this is because, if the total amount of the acid acceptors (hydrotalcite and MgO) falls within the aforementioned range, generation of rust on a mold or the like can be suppressed, and defects that raw materials themselves turn into a white-spotted unwanted object can be reduced.

The medical rubber composition of the present disclosure may contain a co-crosslinking agent. The co-crosslinking agent is preferably a polyfunctional (meth)acrylate compound. The polyfunctional (meth)acrylate compound is more preferably a difunctional or higher-functional (meth)acrylate-based compound and further preferably a trifunctional or higher-functional (meth)acrylate-based compound. Meanwhile, the polyfunctional (meth)acrylate compound is preferably an octafunctional or lower-functional (meth)acrylate-based compound and more preferably a hexafunctional or lower-functional (meth)acrylate-based compound. Examples of the difunctional or higher-functional (meth)acrylate compound can include a compound having at least two acryloyl groups and/or methacryloyl groups. The term "(meth)acrylate" means "acrylate" and/or "methacrylate".

Examples of the difunctional or higher-functional (meth)acrylate-based compound can include di(meth)acrylate of polyethylene glycol, 1,4-butanediol di(meth)acrylate, 1,6-hexanediol di(meth)acrylate, 1,9-nonanediol di(meth)acrylate, trimethylolpropane tri(meth)acrylate, trimethylolethane tri(meth)acrylate, pentaerythritol tri(meth)acrylate, pentaerythritol tetra(meth)acrylate, glycerin tri(meth)acrylate, dipentaerythritol tri(meth)acrylate, dipentaerythritol tetra(meth)acrylate, dipentaerythritol penta(meth)acrylate, dipentaerythritol hexa(meth)acrylate, tripentaerythritol tetra(meth)acrylate, tripentaerythritol penta(meth)acrylate, tripentaerythritol hexa(meth)acrylate, tripentaerythritol hepta(meth)acrylate, and the like.

These co-crosslinking agents may be used singly, or two or more of these co-crosslinking agents may be used in combination.

In the medical rubber composition of the present disclosure, a (d) filler may be blended. Examples of the (d) filler include inorganic fillers such as silica, clay, and talc. The filler is further preferably clay or talc. The filler has a function of adjusting the rubber hardness of the medical rubber part and functions also as an extender for reducing manufacturing cost for the medical rubber part.

Examples of the clay can include calcined clay and kaolin clay. Specific examples of the clay include SILLITIN (registered trademark) Z manufactured by Hoffmann Mineral GmbH, SATINTONE (registered trademark) W manufactured by Engelhard Corporation, NN kaolin clay manufactured by Tsuchiya Kaolin Industry Co., Ltd., PoleStar200R manufactured by Imerys Specialties Japan Co., Ltd., and the like.

Specific examples of the talc include High toron A manufactured by Takehara Kagaku Kogyo Co., Ltd., MICRO ACE (registered trademark) K-1 manufactured by Nippon Talc Co., Ltd., MISTRON (registered trademark) Vapor manufactured by Imerys Specialties Japan Co., Ltd., and the like.

In the medical rubber composition of the present disclosure, a colorant such as titanium oxide or carbon black, polyethylene glycol as a processing aid or as a crosslinking activator, a plasticizer (for example, paraffin oil), and the like may further be blended in appropriate proportions.

The medical rubber composition of the present disclosure is obtained by kneading the (a) base polymer containing the halogenated isobutylene-isoprene rubber, the (b) polyethylene, the (c) triazine derivative as a crosslinking agent, and other blending materials to be added as necessary. The kneading can be performed by using, for example, an open roll, a sealed-type kneader, or the like. The kneaded product is preferably molded in the shape of a ribbon, the shape of a sheet, the shape of a pellet, or the like, and is more preferably molded in the shape of a sheet.

If the kneaded product having the shape of a ribbon, the shape of a sheet, or the shape of a pellet is press-molded, a medical rubber part having a desired shape is obtained. A crosslinking reaction in the medical rubber composition progresses during the pressing. The temperature in the molding is, for example, preferably not lower than 130°C and more preferably not lower than 140°C. Meanwhile, the temperature is preferably not higher than 200°C and more preferably not higher than 190°C. The time for the molding is preferably not shorter than 2 minutes and more preferably not shorter than 3 minutes. Meanwhile, the time is preferably not longer than 60 minutes and more preferably not longer than 30 minutes. The pressure for the molding is preferably not lower than 0.1 MPa and more preferably not lower than 0.2 MPa. Meanwhile, the pressure is preferably not higher than 10 MPa and more preferably not higher than 8 MPa.

Unnecessary portions are cut off and removed from the molded product after the press-molding, such that the molded product has a predetermined shape. The obtained molded product is cleaned, dried, and packaged to manufacture the medical rubber part.

In addition, a resin film may be stacked on and integrated with the medical rubber part. Examples of the resin film include films made from inactive resins such as polytetrafluoroethylene (PTFE), tetrafluoroethylene-ethylene copolymer (ETFE), modified products thereof, and ultrahigh-molecular-weight polyethylene (UHMWPE).

The resin film only has to be, for example, press-molded in a state of being superposed on the rubber composition having the shape of a sheet such that the resin film is integrated with the medical rubber part formed after the press-molding.

EXAMPLES

Hereinafter, the present disclosure will be described in detail by means of examples, but the present disclosure is not limited to the following examples, and any of modifications and implementation modes made within the scope of the gist of the present disclosure is included in the scope of the present disclosure.

[Preparation Of Medical Rubber Compositions]

Rubber compositions were each prepared by blending components other than the crosslinking component among the components indicated in Table 1, kneading the resultant mixture with use of a 10-L pressurization-type sealed kneader at a filling rate of 75%, aging the kneaded product at room temperature, then, adding the crosslinking component to the kneaded product, and kneading the resultant mixture with use of an open roll.

[Manufacturing Of Medical Rubber Plugs]

Each of the aforementioned rubber compositions was molded in the shape of a sheet, sandwiched between an upper mold portion and a lower mold portion, and press-molded in a vacuum at 180°C for 10 minutes. Consequently, a plurality of rubber plugs of vials for a freeze-dried injectable were continuously formed on the above one sheet. In each rubber plug, a flange had a diameter of 19.0 mm, a leg portion had a diameter of 13.2 mm, and a flange piercing portion had a thickness of 2.5 mm. Thereafter, a silicone-based lubricating coat agent was applied on both surfaces of the above sheet. Then, rubber plugs were manufactured through an outer appearance inspection step, a stamping step, a cleaning step, a sterilizing step, and a drying step. Each of the manufactured rubber plugs was used in an eluting material test and a tack test.

[Table 1]

| Medical rubber part sterilization treatment No. | | 1 | 2 | 3 | 4 | 5 | 6 | 7 |
|---|---|---|---|---|---|---|---|---|
| Blending amount (parts by mass) | Chlorinated isobutylene-isoprene rubber | 100 | 100 | 100 | 100 | 100 | 100 | 100 |
| | Polyethylene 1 | 5 | 5 | 5 | 10 | 10 | 0 | 0 |
| | Polyethylene 2 | - | - | - | - | 5 | 10 | - |
| | Triazine derivative | 1.5 | 1.5 | 1.5 | 1.5 | 1.5 | 1.5 | 1.5 |
| | Talc | 50 | 50 | 50 | 50 | 50 | 50 | 50 |
| | Hydrotalcite | 2 | 2 | 2 | 2 | 2 | 2 | 2 |
| | Magnesium oxide | 3 | 3 | 3 | 3 | 3 | 3 | 3 |
| | Carbon black | 0.5 | 0.5 | 0.5 | 0.5 | 0.5 | 0.5 | 0.5 |
| | Titanium oxide | 3 | 3 | 3 | 3 | 3 | 3 | 3 |
| | Oil | 0.5 | 0.5 | 0.5 | 0.5 | 0.5 | 0.5 | 0.5 |
| Before irradiation | Internal environment of packaging article | Oxygen adsorber contained | Nitrogen | Air | Oxygen adsorber contained | Oxygen adsorber contained | Oxygen adsorber contained | Oxygen adsorber contained |
| | Oxygen concentration in packaging article (%) | 0.1% | 3% | 21% | 0.1% | 0.1% | 0.1% | 0.1% |
| I. Eluting material test | Before irradiation | Pass | Pass | Pass | Pass | Pass | Pass | Pass |
| | After 30-kGy irradiation | Pass | Pass | Pass | Pass | Pass | Pass | Pass |
| II. TOC test | After 30-kGy irradiation (relative evaluation of post-irradiation value with respect to pre- irradiation value) | 127% | 140% | 300% | 123% | 119% | 115% | 172% |
| | Evaluation | Slightly good | Slightly good | Bad | Slightly good | Good | Good | Bad |
| III. Tack test | After 30-kGy irradiation (relative evaluation of post-irradiation value with respect to pre- irradiation value) | 113% | 120% | 296% | 114% | 93% | 86% | 154% |
| | Evaluation | Good | Good | Bad | Good | Good | Good | Bad |

(continued)

| Medical rubber part sterilization treatment No. | 1 | 2 | 3 | 4 | 5 | 6 | 7 |
|---|---|---|---|---|---|---|---|
| Comprehensive determination as to suitability for being RTU | Suitable | Suitable | Unsuitable | Suitable | Suitable | Suitable | Unsuitable |

Details of the blending materials used are as follows.

Butylated rubber: HT-1066 (chlorine content:1.26% by weight) manufactured by Exxon Mobil Corporation
Polyethylene 1: MIPELON XM-220 (degree of crystallinity: 69%) manufactured by Mitsui Chemicals, Inc.
Polyethylene 2: Flo-thene UF20S (degree of crystallinity: 35%) manufactured by SUMITOMO SEIKA CHEMICALS CO., LTD.
Triazine derivative: ZISNET DB manufactured by SANKYO KASEI CO., LTD.
Talc: MISTRON Vapor manufactured by Imerys Specialties Japan Co., Ltd.
Hydrotalcite: ALCAMIZER 1 manufactured by Kyowa Chemical Industry Co., Ltd.
Magnesium oxide: MAGSARAT 150s manufactured by Kyowa Chemical Industry Co., Ltd.
Carbon black: DIABLACK G manufactured by Mitsubishi Chemical Holdings Corporation
Titanium oxide: KR-380 manufactured by Titan Kogyo Ltd.
Oil: PW380 manufactured by Idemitsu Kosan Co., Ltd.

[Evaluation Methods]

(1) Measurement Of Melting Heat Quantity Of Polyethylene

The melting heat quantity of each polyethylene was acquired from a primary test at elevated temperatures through differential scanning calorimetry (DSC).
DSC measurement condition: 20°C to 200°C, with a rate of temperature elevation being 10°C/min.

(2) Eluting material Test

Measurement sample: in a state where each medical rubber plug was put in a polyethylene bag (in packaging modes shown in FIG. 1 and FIG. 2, and in an internal environment of the packaging article indicated in Table 1), the medical rubber plug was irradiated with the gamma ray so as to have an absorbed dose of 30 kGy, whereby a rubber plug having been irradiated with the gamma ray was obtained.
The measurement sample was tested according to the method in "Extractable substances" described in "7.03 Test for Rubber Closure for Aqueous Infusions" of the 17th edition of the Japanese Pharmacopoeia. Conditions of passing the test were as follows.

Properties of test solution: colorless and clear
Ultraviolet transmissivity: a transmissivity being not lower than 99.0% at each of a wavelength of 430 nm and a wavelength of 650 nm with a layer length of 10 mm
Ultraviolet absorption spectrum: an absorbance being not higher than 0.20 at a wavelength of 220 nm to 350 nm
pH: the difference between the test solution and a blank test solution being not larger than 1.0
Zinc: the absorbance of a sample solution being not higher than the absorbance of a standard solution
Potassium permanganate reducing substance: not higher than 2.0 mL/100 mL (according to a standard in the Japanese Pharmacopoeia)
Post-evaporation residue: not larger than 2.0 mg

If any of these conditions was not satisfied, the rubber plug was evaluated as "Not pass". Meanwhile, if all of the conditions were satisfied, the rubber plug was evaluated as "Pass".

(3) TOC Test

Regarding the eluting liquid subjected to the eluting material test in "(2)", a total organic carbon value TOC (NPOC: TOC obtained through acidification-aeration treatment) was measured.

Measurement analysis device: Shimadzu total organic carbon analyzer TOC-VCSH (of a combustion oxidizing type)
Measurement analysis condition: a combustion tube temperature being 680 degrees with use of a high-sensitivity catalyst
Carrier gas: highly purified air at 150 mL/min.
Injection amount: 200 μL
Concentration of added acid: 1.5%
Aeration treatment time: 90 sec.

Non-elution characteristics before and after irradiation with the gamma ray were evaluated. The TOC after irradiation with the gamma ray was evaluated by being indicated as a relative index with the TOC before irradiation with the gamma ray being regarded as 100%. A larger numeral means that the non-elution characteristics has deteriorated more relative to the non-elution characteristics before irradiation with the gamma ray.

Evaluation criteria
Good: not higher than 120% (approximately equal to a pre-irradiation value)
Slightly good: higher than 120% and not higher than 150% (having slightly deteriorated relative to the pre-irradiation value)
Bad: higher than 150% (having deteriorated to a large extent, relative to the pre-irradiation value)

(4) Tack Test

A tack test was performed as follows by using a desktop tester EZ-SX available from Shimadzu Corporation. As shown in FIG. 3, a sample 13 was fixed to a fixation jig 15 on the lower side, a metal probe 17 on the upper side was pressed onto the sample 13, and the pressed state was maintained for 10 seconds after the pressure had reached a set value. Thereafter, the metal probe 17 was lifted upward, and a peak value of close contact force generated between the metal probe 17 and the sample 13 was used as a tack value. The measurement was performed 5 times on each sample. From among the measurement values obtained as a result, the maximum value and the minimum value were excluded, and the average value of the remaining three measurement values was calculated. The close contact force of the sample having been irradiated with the gamma ray was indicated as an index with the close contact force of the sample, which had not yet been irradiated with the gamma ray, being regarded as 100. A smaller index indicates a lower tackiness and a more favorable result.

Measurement conditions

Pressing speed: 0.5 mm/s
Pressing load: 1000 g of weight
Pressed-state maintaining time: 10 seconds
Pull-up speed: 10 mm/s
Ultimate pull-up distance: 3 mm
Diameter of probe: 10 mm
Evaluation criteria
Good: not higher than 120% (approximately equal to a pre-irradiation value)
Slightly good: higher than 120% and not higher than 150% (having slightly deteriorated relative to the pre-irradiation value)
Bad: higher than 150% (having deteriorated to a large extent, relative to the pre-irradiation value)

The results of the eluting material test, the TOC test, and the tack test are indicated together in Table 1.

Determination as to suitability for being ready-to-use was made as follows.

If the result of the eluting material test was "Pass", the result of the TOC test was "Slightly good" or the more favorable evaluation result, and the result of the tack test was "Slightly good" or the more favorable evaluation result, it was determined that suitability for being ready-to-use was attained. Meanwhile, if any of the evaluation results was unfavorable, it was determined that suitability for being ready-to-use was not attained.

According to the results in Table 1, the sterilization method leads to obtainment of a medical rubber part in which non-elution characteristics are maintained and which experiences less troubles in a manufacturing process for the medical product. The sterilization method includes irradiating, with gamma ray, a packaging article for a medical rubber part made from an elastomer that contains a polyethylene, the packaging article accommodating a plurality of the medical rubber parts.

The present disclosure makes it possible to provide a sterilization method for a medical rubber part in which non-elution characteristics are maintained even after sterilization with gamma ray and which experiences less troubles in a manufacturing process for the medical product.

A sterilization method for a medical rubber part according to aspect (1) of the present disclosure includes irradiating, with gamma ray, a packaging article for a medical rubber part made from an elastomer that contains a polyethylene, the packaging article accommodating a plurality of the medical rubber parts.

A sterilization method for a medical rubber part according to aspect (2) of the present disclosure is the sterilization

method for the medical rubber part according to aspect (1) of the present disclosure, the sterilization method including irradiating, with the gamma ray, the packaging article having an oxygen concentration not higher than 5%.

A sterilization method for a medical rubber part according to aspect (3) of the present disclosure is the sterilization method for the medical rubber part according to aspect (2) of the present disclosure, wherein the packaging article having an oxygen concentration not higher than 5% is a packaging article in which nitrogen is sealed.

A sterilization method for a medical rubber part according to aspect (4) of the present disclosure is the sterilization method for the medical rubber part according to aspect (2) of the present disclosure, wherein the packaging article having an oxygen concentration not higher than 5% is a packaging article in which an oxygen adsorber is sealed.

A sterilization method for a medical rubber part according to aspect (5) of the present disclosure is the sterilization method for the medical rubber part according to any one of aspects (1) to (4) of the present disclosure, the sterilization method including performing irradiation with the gamma ray such that an absorbed dose of the gamma ray is not lower than 15 kGy.

A sterilization method for a medical rubber part according to aspect (6) of the present disclosure is the sterilization method for the medical rubber part according to any one of aspects (1) to (5) of the present disclosure, wherein the elastomer is a cured product of a rubber composition that contains: a (a) base polymer containing a halogenated isobutylene-isoprene rubber; a (b) polyethylene; and a (c) triazine derivative as a crosslinking agent.

A sterilization method for a medical rubber part according to aspect (7) of the present disclosure is the sterilization method for the medical rubber part according to aspect (6) of the present disclosure, wherein the halogenated isobutylene-isoprene rubber is at least one rubber selected from the group consisting of chlorinated isobutylene-isoprene rubber, brominated isobutylene-isoprene rubber, and brominated isobutylene-para-methylstyrene copolymer rubber.

A sterilization method for a medical rubber part according to aspect (8) of the present disclosure is the sterilization method for the medical rubber part according to any one of aspects (1) to (7) of the present disclosure, wherein the (b) polyethylene contains a polyethylene having a degree of crystallinity not higher than 70%.

A sterilization method for a medical rubber part according to aspect (9) of the present disclosure is the sterilization method for the medical rubber part according to any one of aspects (1) to (8) of the present disclosure, wherein an amount of the (b) polyethylene contained per 100 parts by mass of the (a) base polymer is not smaller than 3 parts by mass and not larger than 30 parts by mass.

A sterilization method for a medical rubber part according to aspect (10) of the present disclosure is the sterilization method for the medical rubber part according to any one of aspects (1) to (9) of the present disclosure, wherein the medical rubber part is a rubber plug for a vial, a cap or a plunger stopper for a syringe, or a rubber plug for a vacuum blood collection tube.

## Claims

1. A sterilization method for a medical rubber part (1), the sterilization method comprising
irradiating, with gamma ray, a packaging article (3, 5, 7) for a medical rubber part (1) made from an elastomer that contains a polyethylene, the packaging article (3, 5, 7) accommodating a plurality of the medical rubber parts (1).

2. The sterilization method for the medical rubber part (1) according to claim 1, the sterilization method comprising
irradiating, with the gamma ray, the packaging article (3, 5, 7) having an oxygen concentration not higher than 5%.

3. The sterilization method for the medical rubber part (1) according to claim 2, wherein
the packaging article (3, 5, 7) having an oxygen concentration not higher than 5% is a packaging article in which nitrogen is sealed.

4. The sterilization method for the medical rubber part (1) according to claim 2, wherein
the packaging article (3, 5, 7) having an oxygen concentration not higher than 5% is a packaging article in which an oxygen adsorber (11) is sealed.

5. The sterilization method for the medical rubber part (1) according to any one of claims 1 to 4, the sterilization method comprising
performing irradiation with the gamma ray such that an absorbed dose of the gamma ray is not lower than 15 kGy.

6. The sterilization method for the medical rubber part (1) according to any one of claims 1 to 5, wherein
the elastomer is a cured product of a rubber composition that contains:

a (a) base polymer containing a halogenated isobutylene-isoprene rubber;

a (b) polyethylene; and
a (c) triazine derivative as a crosslinking agent.

**7.** The sterilization method for the medical rubber part (1) according to claim 6, wherein the halogenated isobutylene-isoprene rubber is at least one rubber selected from the group consisting of chlorinated isobutylene-isoprene rubber, brominated isobutylene-isoprene rubber, and brominated isobutylene-para-methylstyrene copolymer rubber.

**8.** The sterilization method for the medical rubber part (1) according to any one of claims 1 to 7, wherein the (b) polyethylene contains a polyethylene having a degree of crystallinity not higher than 70%.

**9.** The sterilization method for the medical rubber part (1) according to any one of claims 1 to 8, wherein an amount of the (b) polyethylene contained per 100 parts by mass of the (a) base polymer is not smaller than 3 parts by mass and not larger than 30 parts by mass.

**10.** The sterilization method for the medical rubber part (1) according to any one of claims 1 to 9, wherein the medical rubber part (1) is a rubber plug for a vial, a cap or a plunger stopper for a syringe, or a rubber plug for a vacuum blood collection tube.

FIG. 1

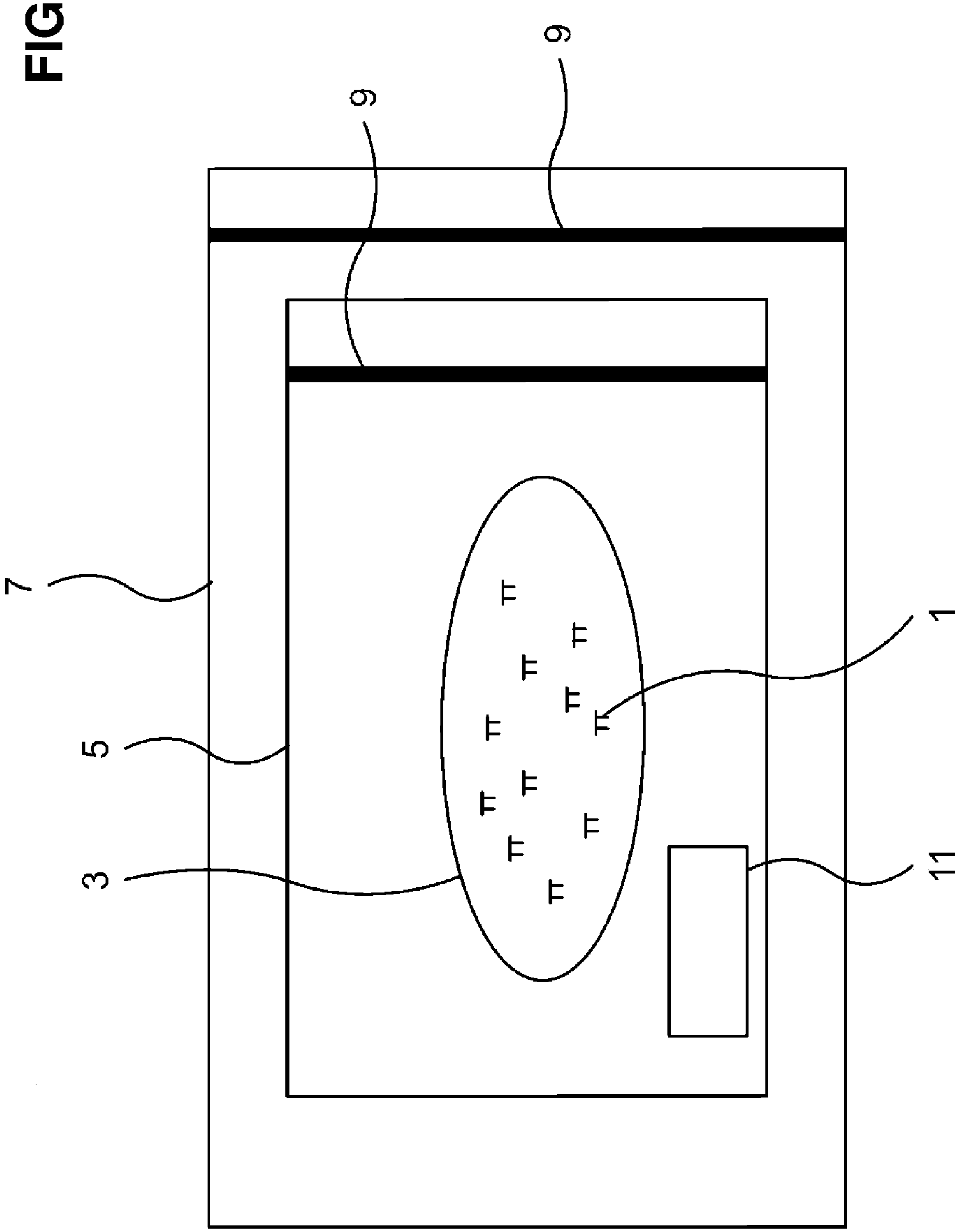

FIG. 2

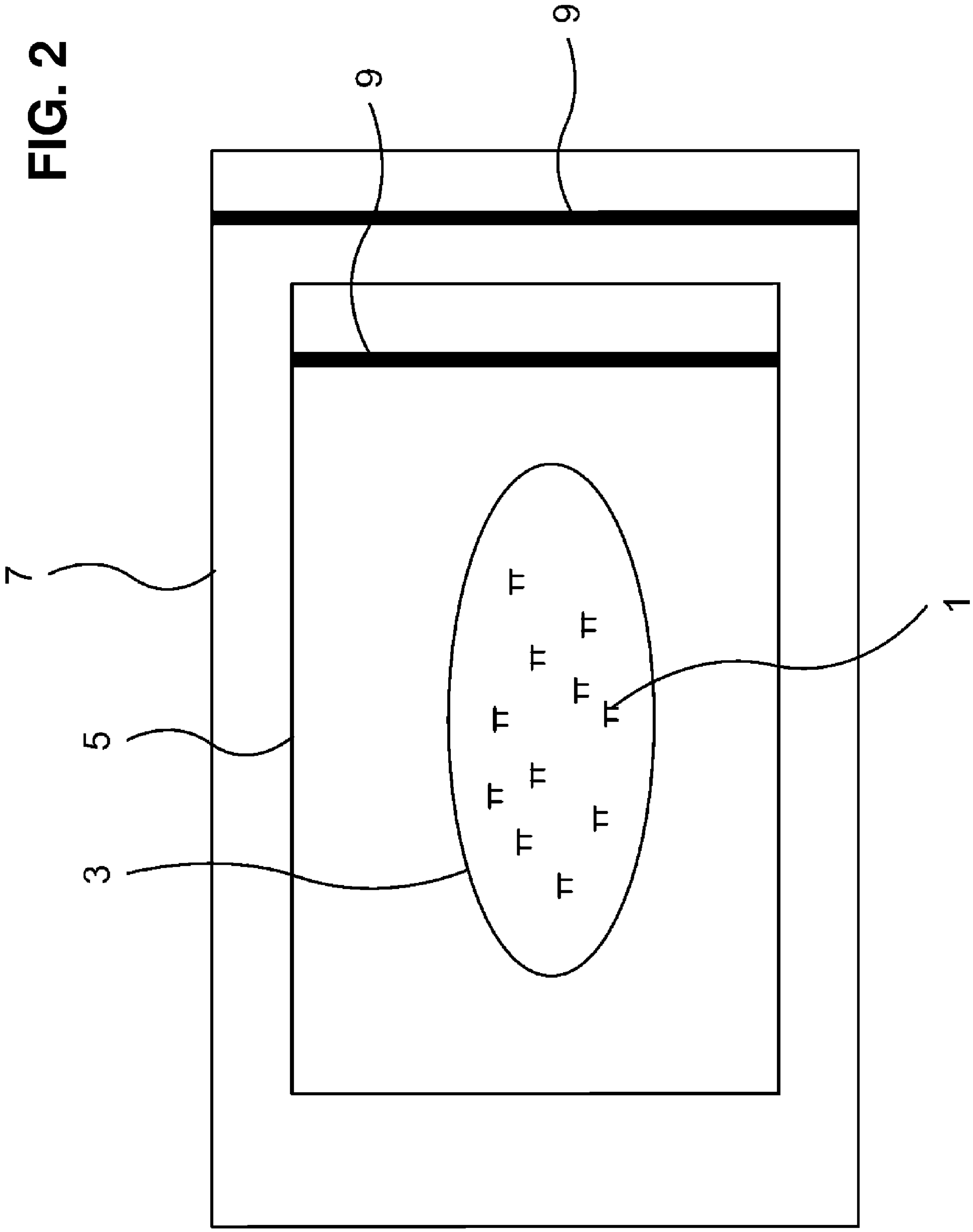

FIG. 3

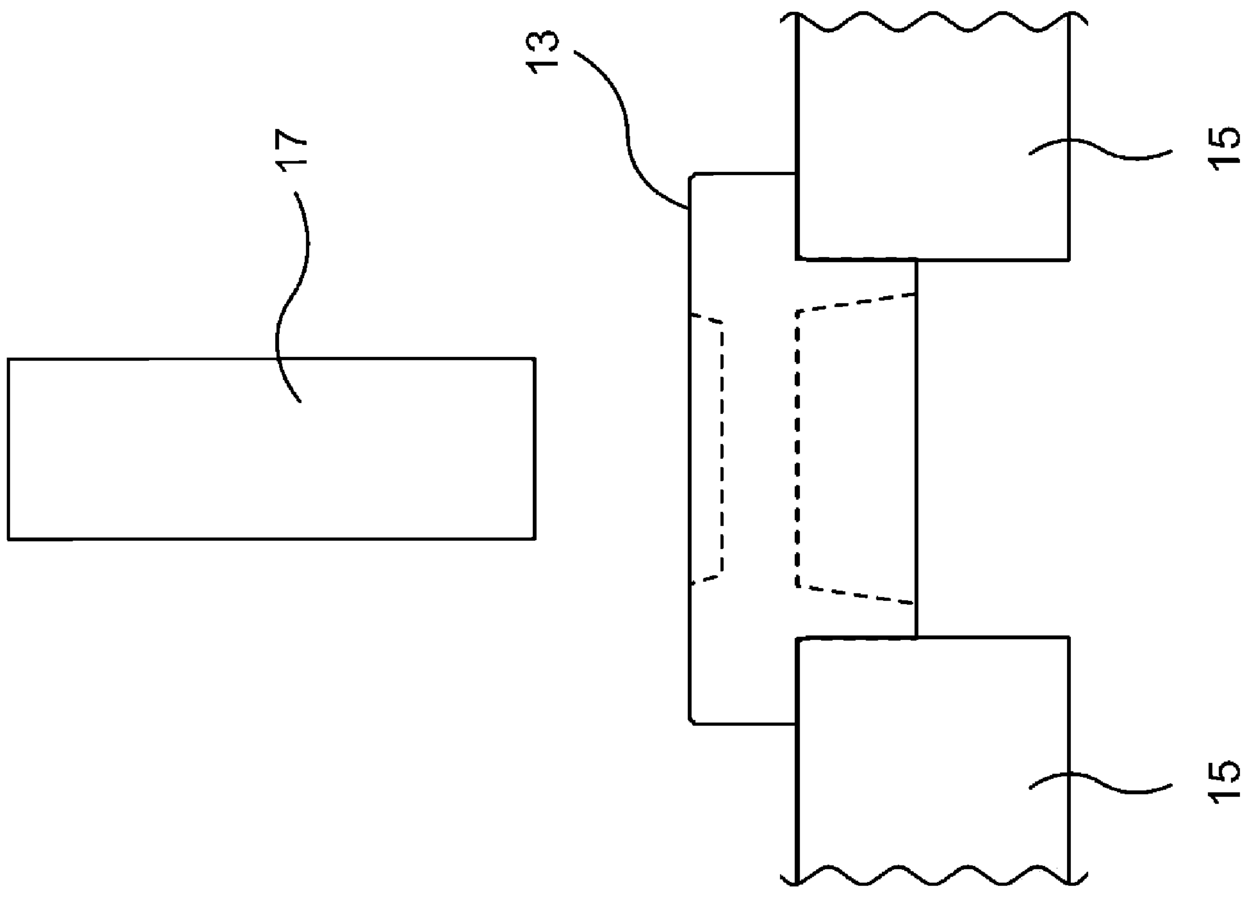

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H10179690 A
- JP 2002301133 A
- JP 2017531604 W


### Non-patent literature cited in the description


- Test for Rubber Closure for Aqueous Infusions. Japanese Pharmacopoeia